(19)

Europäisches
Patentamt
European
Patent Office
Office européen
des brevets

(11) **EP 2 461 162 A1**

(12) **EUROPEAN PATENT APPLICATION**

(43) Date of publication:
06.06.2012 Bulletin 2012/23

(51) Int Cl.:
*G01N 33/569* (2006.01)

(21) Application number: 10306347.5

(22) Date of filing: 03.12.2010

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Texcell, .
91000 Evry (FR)**

(72) Inventor: **Dorange, Fabien
28630, Nogent Le Phaye (FR)**

(74) Representative: **Warcoin, Jacques
Cabinet Regimbeau
20, rue de Chazelles
75847 Paris Cedex 17 (FR)**

(54) **Method for determining the titre of viruses by using infectious standards**

(57)     The present invention is directed to a titration method for cytopathogenic agent, particularly infectious viruses. In particular, the present invention is based on a new method for *in vitro* determining the titre of infectious agent, such as viruses in solution by comparing the viability of the infected cell monolayer against a standard curve obtained by measuring the cell viability of monolayers infected with a known cytopathogenic agent stock. The invention is of special utility in the pharmaceutical industry for cytopathogenic agent clearance studies and for the identification and optimization of antiviral compounds involved in drug development and/or in pharmaceutical compositions.

EP 2 461 162 A1

**Description**

The present invention is directed to a titration method for cytopathogenic agent, such as infectious viruses. The present invention could be applied to any infectious agents that induce an effect in cells that could be quantifiable by the use of a marker, preferably a cell viability marker, including metabolites, reporter protein, or agent's components. In particular, the present invention is based on a new method for *in vitro* determining the titre of cytopathogenic agent in solution by comparing the viability of the infected cell monolayer against a standard curve obtained by measuring the cell viability of monolayers infected with a known cytopathogenic agent stock. The invention is of special utility in pharmaceutical and biotechnological industries for cytopathogenic agent clearance studies and for the identification and optimization of anti-cytopathogenic agent compounds involved in drug development and/or in pharmaceutical compositions.

Virus titration is a method used in several fields in virology:

- Vaccine production and gene therapy when using virus or viral vector.
- Recombinant protein expression when using viral expression system in order to define the multiplicity of infection for maximal protein expression (baculovirus, semliki forest virus).
- Viral clearance studies in order to estimate the inactivation/removal of viruses during manufacturing process.

The methods most commonly used for the determination of virus titre are plaque assays and endpoint dilution assays (Kaplan M. & Koprowski H., 1973; Schwartz D., 1993).

Plaques assays and end point dilution assays are gold standards methods to perform the titration of viruses. However these methods are labor intensive (preparation of the cell monolayers one day before the titration, add of agarose or CMC monolayer for plaque assays ("CMC" for carboxymethylcellulose) and are time and reagent-consuming (in general 7 days to complete, require high virus supernatants and cellular volumes to handle, and high number of replicates). They need highly trained technicians and are difficult to adapt for automatisation and high throughput.

Some improvements of these methods were assessed, such as the use of MTT (3-(4,5-Dimethylthiazol-2-yl)-2,5-diphenyltetrazolium bromide) (Mena et al, 2003) or immunodetection methods to facilitate the readings of infected versus non-infected wells (Kitts PA & Green G). But in any cases, these improvements don't overcome the major drawbacks of gold standard methods.

Several recent methods were developed in order to prevent such limitations of gold standards assays.

Due to the development of these new methods, methods were divided into two groups: cell-based assays (including plaque assays and end-point dilution assays) and direct assays. Cell-based assays are infectious assays that determine the number of infectious viruses and are also qualitative and quantitative methods, whereas direct assays determine the number of a virion component (most commonly the viral genome) that can not be directly correlated to an infectious state and is also defined as a quantitative method.

In cell-based methods, immunodetection methods using flow cytometry have been successfully applied for the determination of virus titre by detecting viral proteins in infected cells (Mulvania et al., 2004). Immunodetection assays are rapid methods when performed with a flow cytometer, but limitations are link to the use of specific antibodies for each virus that could show lot by lot variation. This method needs expensive reagent and specialized equipment, and as being recognized less sensitive than plaque assays (Lambeth et al, 2005).

Reporter genes have been incorporated in engineered viruses to titrate viruses by detecting the expression of their reporter proteins like GFP (Green Fluorescent Protein) or β-galactosidase (Cha et al., 1997; Yahata et al., 2000). These recombinant viruses could however suffer in their use in viral clearance studies validation for pharmaceutical process and in virucidal studies as their modifications could affect their main characteristics (structure, isoelectric charge).

Determination of virus titre was reported with measurement of cell viability by using Alamar blue in cell growth assay (Pouliquen et al., 2006). The authors define a good correlation between cell growth and baculovirus virus titre. However informations for mammalian viruses are not available and titre determination is in the limited range of 5.1E+06 and 2.8E+08 PFU/ml. The authors recommend 2 steps titration methods for out of range virus titre stock with the dilution of higher titre stocks and the use of alternative methods for lower titres.

Another cell based assay was reported with the estimation of virus titre based on viable cell size (Janakiraman et al., 2006). The cell size assay is a rapid method but this method could only be used with viruses that induce a size change in infected cells.

In direct methods, QPCR was used to determine viral titres (Lo and Chao, 2004). Flow cytometry was used to determine viral titres with the use of SYBR Green (asymmetrical cyanine dye used as a nucleic acid stain in molecular biology) to stain the virus particles (Shen et al., 2002).

As no fully satisfactory method was described, we developed a method that possesses the following characteristics:

- Infectious assay
- Quick
- Ease of results interpretation
- Universal methods (methods and reagents used are identical among different viruses).
- Range from 1E+02 to 1E+06 $TCID_{50}$/ml
- Automation
- High throughput

The following Table 1 presents the characteristics of actual method.

Table 1

|  |  | Infectious assays | quick | Ease of results interpretation | Universal methods | Range from $10^2$ to $10^6$ TCID50/ml | automatisation | high throughput |
|---|---|---|---|---|---|---|---|---|
| Plaque assay |  | ✓ | ☐ | ☐ | ✓ | ✓ | ☐ | ☐ |
| End point dilution |  | ✓ | ☐ | ☐ | ✓ | ✓ | ☐ | ☐ |
| immunodetection assay | flow cytometry | ✓ | ✓ | ✓ | ☐ | ☐ | ✓ | ✓ |
|  | microscopy examination | ✓ | ☐ | ☐ | ☐ | ✓ | ☐ | ☐ |
| cell viability assay | MTT in end point dilution assay | ✓ | ☐ | ✓ | ✓ | ✓ | ☐ | ☐ |
|  | Alamar blue in cell viability assay | ✓ | ✓ | ✓ | ☐ | ☐ | ✓ | ✓ |
| cell size assay |  | ✓ | ✓ | ✓ | ☐ | ✓ | ✓ | ✓ |
| real time PCR |  | ☐ | ✓ | ✓ | ☐ | ✓ | ✓ | ✓ |
| sybrgreen assay |  | ☐ | ✓ | ✓ | ✓ | ☐ | ✓ | ✓ |

There is a need, therefore, to provide a method to determine the virus, load of a batch cell culture.

There is a need for monitoring methods that can give rapid results, rather than the labor-intensive and time-consuming methods currently employed for that purpose.

This is the object of the present invention.

The present invention was developed to circumvent the limitations of actual methods as no methods possess all the characteristics needed.

The method of the present invention, named "titration by comparison with infectious standards", is a viability cell based assay.

In a first aspect the present invention is directed to the determination of the infectious titre of a cytopathogenic agent solution, such as viral solution, by comparison with a standard curve of a known cytopathogenic agent stock. In the method of the present invention, the step of titration is based on the determination of cytopathogenic agent titres by measuring the metabolic activities of the infected cell monolayer by using a cell viability marker (ATP (Adenosine-5'-triphosphate), LDH (Lactate deshydrogenase).

In a second aspect, the present invention relates to a method for the screening, the identification or the optimization of anti -cytopathogenic agent test compounds involved in drug development and/or in pharmaceutical compositions, said method comprising the cytopathogenic agent titration method of the first aspect of the present invention.

In a third aspect, the present invention is directed to a method for evaluating the capacity of industrial process to eliminate or inactivate cytopathogenic agent and for the determination of their reduction factor.

Use of sensitive reagents for the measure of cell viability in absorbance, fluorescence and luminescence present an accurate quantification of viable cell number. The cytopathogenic agent quantification is done by measuring cell viability and by comparing it with a cell viability standard curve of infected cell monolayer.

For the first time, the inventors have demonstrated a correlation between the viability measured in a cell monolayer and the infectious cytopathogenic agent load with a low detection limit, for example for viral infection around 1E+02 $TCID_{50}$/ml ($TCID_{50}$ for median Tissue Culture Infective Dose (quantity of a cytopathogenic agent (virus) which will produce a cytopathic effect in 50 % of the cultures inoculated))

The reagent used is a marker of the cell viability and can be used to measure the viability of any cell line.

The method of the present invention is an universal titration method that could be applied for any human and animal viruses. This method can be easily automated and can be used in high throughput assay.

The method described in the present patent has been developed with viruses and can be extended to any transmissible agent causing cytopathogenic effect on target cells.

The method of the present invention resolves the actual methods limitations and answers to all characteristics.

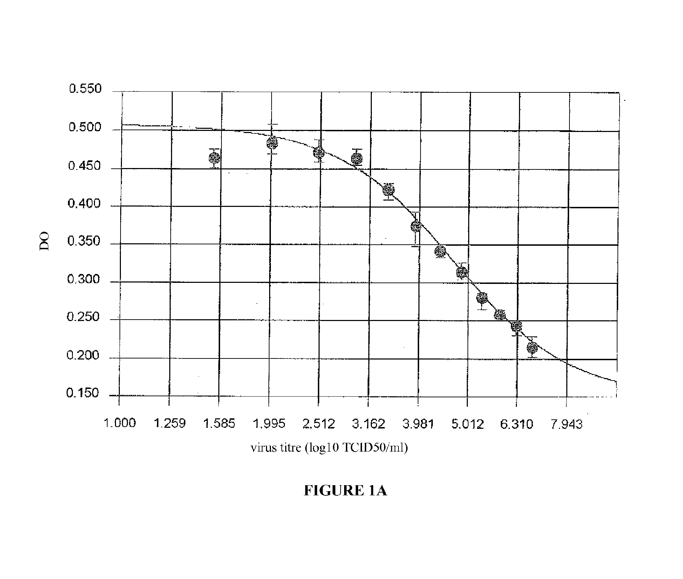

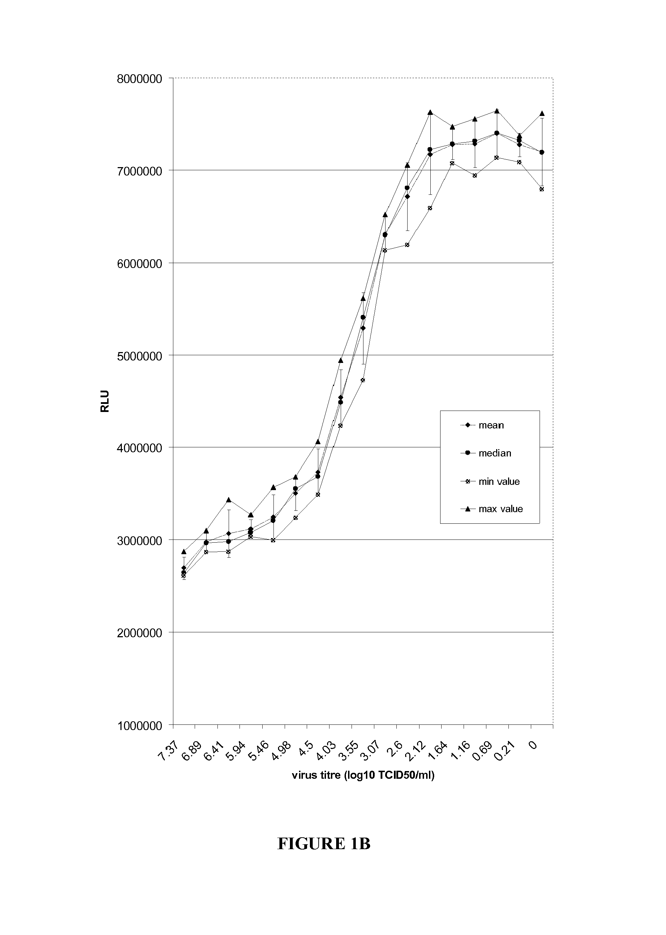

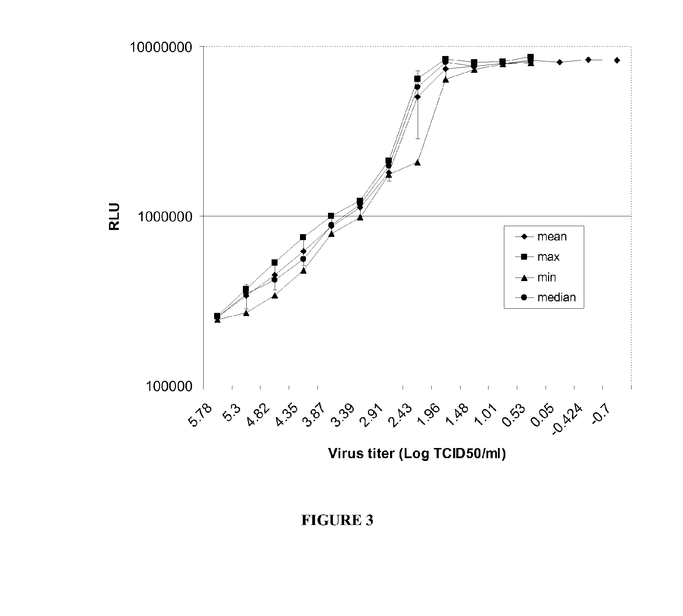

- Infectious assay: Titration are performed on sensitive cell lines and also measured the infectious potential of cytopathogenic agent, such as viruses.
- Quick: This titration method is timeliness. For example, for viral infection, titre determination can be performed in less than four days depending of the virus instead of usually seven days in gold standard methods.
- Ease of results interpretation: Results are obtained by reading 96 wells plate on a luminometer. Data interpretation software could be link to the reader.
- Universal method: the method could be used for any cytopathogenic agent, such as replicative human, animal and bacterial viruses. Figures 1A and 1B and 3 present standards curves obtained with the Minute Virus of Mice (MVM) and Pseudorabies virus (PRV) on A9 and RK13 cell lines respectively.
- Range from 1E+02 to 1E+06 $TCID_{50}$/ml: Figures 1A, 1B and 3 present standards curves obtained with the Minute Virus of Mice and Pseudorabies virus (PRV) on A9 and RK13 cell lines respectively. The sigmoid curves obtained allow the determination of titre from approximately 1E+06 to 1E+02 $TCID_{50}$/ml for both viruses.
- Automation: Liquid handling, dilution, cells and reagents distribution and reading could be automated.
- High throughput could be easily performed as in only one 96 wells plate, several cytopathogenic agent stocks could be titrated. At least 30 wells could be attributed to standards from 1E+06 to 1E+01 $TCID_{50}$/ml for example of a known viral stock with triplicates 3 fold dilutions. This leads to 66 free wells that could be used for the titration in triplicate of 22 unknown stocks without testing different dilutions.

Thus, the present invention is directed to a cell viability-based method for in-process monitoring cytopathogenic agent titration of cultured infected host cells with said cytopathogenic agent, encompassing the steps of:

a) obtaining from a host cell culture infected with said cytopathogenic agent a concentration-dependent plurality of samples;
b) determining the level of the viability of the host cells in the samples by measuring a significant cell viability parameter; and
c) comparing the measure of said parameter with a standard curve obtained in a same condition for a control culture of infected host cell with said cytopathogenic agent.

By "cytopathogenic agent" it is intended to designate any agent capable of infecting host cells, such as infectious virus, including bacteriophage, non-conventional transmissible agent (NCTA) and which are pathogenic for said infected host cell, preferably that are capable of causing a disease.

The term "in-process" refers in the present description to the monitoring of the parameters that are characteristic of cell and cytopathogenic agent culture, including a cytopathogenic agent infected cell culture, throughout the period of the culture. The monitoring can be periodic monitoring wherein samples are withdrawn from the culture at selected time points and for different initial concentrations of the virus containing sample, parameters such as viability are detected and measured and the parameters are plotted versus the time of the culture and the initial concentration of the cytopathogenic agent containing sample ("cytopathogenic agent containing sample" used for infection the host cells at the beginning of the culture).

In another aspect, the present invention provides a method for monitoring a cytopathogenic agent infection or for monitoring the progression of a cytopathogenic agent infection in a cell culture, said method comprising the step of:

a) infecting a host cell population with a cytopathogenic agent;
b) obtaining from said host cell culture infected with said cytopathogenic agent a concentration-dependent plurality of samples;
c) determining the level of the viability of the host cells in the samples by measuring a significant cell viability parameter; and
d) comparing the results or the curve obtained from said concentration-dependent plurality of samples with a standard curve obtained in a same condition for a control culture of virus infected host cell, said comparison being significant of the cytopathogenic agent infection or its progression.

In a preferred embodiment, the standard curve is obtained in a same condition for a control culture of cytopathogenic agent infected host cell in which the host cells are infected with virus or virus particles by contacting the host cells with a portion of a viral preparation having a known titre or concentration.

In a preferred embodiment, the standard curve is obtained in a same condition for a control culture of cytopathogenic agent infected host cell, wherein the cytopathogenic agent used for said control culture is the same virus, or virus having the same replication time and in which the host cells are infected with said virus or virus particles by contacting the host cells with a portion of a cytopathogenic agent preparation having a known titre or concentration.

In a preferred embodiment, the standard curve is obtained in a same condition for a control culture of said cytopathogenic agent infected host cell, wherein a range of a concentration of a cytopathogenic agent preparation having a known titre or concentration is implemented for the control culture.

In a more preferred embodiment, the standard curve is obtained by a method comprising the step of:

a) withdrawing from the culture at a selected time and for different initial concentrations of the cytopathogenic agent containing sample; and

b) determining or measuring a viability parameter of said cytopathogenic agent infected sample and plotting this parameter versus the initial virus concentration of the culture, or the initial concentration of the cytopathogenic agent containing sample used for infecting the host cells at the beginning of the culture.

In a preferred embodiment, said cytopathogenic agent is selected from the group of virus, or virus particle. Viruses are the most preferred of cytopathogenic agents.

In a preferred embodiment, this standard curve is obtained for a selected time of the culture comprised between 24 hours and 150 hours, more preferably between 48 hours and 144 hours, 72 hours $\pm$ 24 hours being the most preferred.

In a preferred embodiment, this standard curve is obtained for a range of initial virus concentration comprised between $5.10^8$ to $0,5$ $TCID_{50}$/ml, preferably $10^8$ to $10^1$, $5.10^7$ to $10^1$' or $10^7$ to $10^1$ $TCID_{50}$/ml.

In a preferred embodiment, this range of concentration is made with 3-fold dilutions of a known initial cytopathogenic agent stocks.

In a preferred embodiment, when said cytopathogenic agent is a virus, one volume, preferably 50 $\mu$l, of the virus infected sample (or standard) is added to one volume of a cell suspension, preferably said cell suspension containing between $10^3$ to $10^5$ host cells, more preferably between $5.10^3$ to $5.10^4$ host cells, $10^4 \pm 2.10^3$ host cells being the most preferred.

In a preferred embodiment the step of determining the level of the viability of the host cells in the samples by measuring a significant cell viability parameter is determined by the measure of the intracellular ATP or the LDH activity of the host cells.

In a more preferred embodiment the step of determining the level of the viability of the host cells in the samples by measuring a significant cell viability parameter is determined by the measure of the intracellular ATP after the lysis of the cytopathogenic agent infected host cells of the sample.

Depending of the host cells used in the present method, the lysis buffer can be adapted to these host cells. For example, anionic, cationic or zwitterionic detergents, DMSO, SDS CHAPS, Triton X-series, such as X-100 can be used for cells lysis.

Detergent cell lysis is a milder and easier alternative to physical disruption of cell membranes, although it is often used in conjunction with homogenization and mechanical grinding. Detergents break the lipid barrier surrounding cells by solubilizing proteins and disrupting lipid:lipid, protein:protein and protein:lipid interactions. Detergents, like lipids, self associate and bind to hydrophobic surfaces. They are comprised of a polar hydrophilic head group and a nonpolar hydrophobic tail and are categorized by the nature of the head group as either ionic (cationic or anionic), nonionic or zwitterionic. Their behavior depends on the properties of the head group and tail.

In a more preferred embodiment the intracellular ATP is determined by bio luminescence.

As used herein, the term "host" refers to any prokaryotic or eukaryotic (e.g. mammalian, insect, yeast, plant, avian, animal, etc.) organism that is a recipient of cytopathogenic agent, such as a replicable virus or virus particle or a NCTA (preferably prion).

Any cells may be used in the methods of the present invention so long as the cells may be infected by the virus of interest, preferably selected from the group consisting of cell lines or primary cells that are permissive to cytopathogenic agent infection

In the various embodiments of this method of the invention, the host cells can be selected from the group consisting of bacterial, insect, plant and animal cell

Representative host cells that may be used according to this aspect of the invention include, but are not limited to animal cells. Preferred animal host cells include mammalian cells, but reptilian or avian cells may be also used.

Particularly preferred are cells from mammalian cell lines, mouse, rat, rabbit, or Primates cell lines, more particularly human and simian, are the most preferred.

Among the cell lines host cells from cell lines that may be used according to this aspect of the invention, particularly for virus as cytopathogenic agent, they include, but are not limited to NIH3T3; 293, CHO, COS, VERO, BHK, BSC-1, Hela, rabbit embryo chondrocytes, HepG2, Hep3B, Lymphoblastoïd cell lines, glial cell lines, Human T cell lines, RK-13 and A9 cell lines.

RK-13 and A9 cell lines are particularly preferred.

These and other suitable host cells are available commercially, for example, from European Collection of Cell Cultures (ECACC) or American Type Culture Collection (ATCC).

In the various embodiments of the methods of the invention, the virus can be selected from the group consisting of any viruses that could lead to a cytopathogenic effect in a cell monolayer leading to cell death.

Although the methods should not be construed as to be applicable solely to the strain of virus, host cells or their combination as indicated below, in the various embodiments of this method of the invention, the virus can be selected from the group comprising poxviridae, orthomyxoviridae, paramyxoviridae picornaviridae, polyomaviridae, herpesviridae, adenoviridae, parvoviridae, reoviridae, coronaviridae, flaviviridae, togaviridae, vesiculovirus, Rhabdoviridae and baculoviridae .

In a preferred embodiment, the viruses or the virus particles are selected from the group consisting of polyomavirus, adenovirus, simplex virus, varicellavirus, parvovirus, orthopoxvirus,- parainfluenza virus, cardiovirus, Enterovirus, hepatovirus, Aphtovirus, paramyxovirus, vesiculovirus, alphavirus, pestivirus, flavivirus, parvovirus, alphabaculovirus.

In another preferred embodiment, the viruses belong to the family of orthomyxoviridae, in particular influenza virus. The influenza virus is selected from the group consisting of human influenza virus, avian influenza virus, equine influenza virus, swine influenza virus, feline influenza virus. Influenza virus is preferably selected in strains A, B and C. Among strains A, one can recite viruses with different subtypes of haemagglutinin and neuraminidase, such as without limitation H1N1, H2N2, H3N2, H4N2, H4N6, H5N1, H5N2, H7N7 et H9N2.

In another preferred embodiment, the viruses belong to the family of paramyxoviridae. Preferably the virus belong to the morbillivirus genus selected in the group comprising measles virus, respirovirus genus selected in the group of Sendai virus and, human para-influenza types I and III, or Pneumovirus genus such as Respiratory Syncythial virus (RSV),

In another preferred embodiment, the viruses, the related viral vectors, the viral particles and vaccines belong to the family of togaviridae. Preferably the virus is an alphavirus selected in the group comprising Sindbis virus and Semliki forest viru.

In another preferred embodiment, the viruses belong to the family of flaviviridae, particularly in the group consisting of the Genus Flavivirus in particular Dengue virus, Japanese encephalitis virus, Yellow fever virus and West Nile virus,Genus Hepacivirus (type Hepatitis C virus) and Genus Pestivirus in particular Bovine viral diarrhea virus.

In another preferred embodiment, the viruses belong to the family of coronaviridae, coronavirus genus in particular transmissible gastroenteritis virus.

In another preferred embodiment, the viruses belong to the family of Picornaviridae, preferably cardiovirus genus selected among Theiler virus, EMCV virus, enterovirus genus selected among Equine rhinovirus (ERV), Human rhinovirus (HRV), poliovirus sabin and poliovirus saukett, and hepatovirus genus such as hepatitis A virus (HAV).

In another preferred embodiment, the viruses or the viral particles belong to the family of Herpesviridae. Preferably the virus is a simplexviruses selected among the group comprising Herpes simplex virus-1 (HSV-1), Herpes simplex virus-2 (HSV-2), and varicellovirus such as Pseudorabies virus (PRV).

In another aspect, the present invention is directed to a method for the screening, the identification or the optimization of antiviral test compounds, preferably these test compounds being involved in drug development and/or in pharmaceutical compositions, said method comprising the step of:

A) Monitoring or determining the viral titration of cultured infected virus host cells, by the method according to the present invention and wherein the viral titration is carried out in presence and in absence of the antiviral compound which is desired to be tested;
B) Comparing the measure of the viral titration obtained and determining whether this antiviral test compound has the capacity to modulate, preferably to decrease the viral titration and, thus, the capacity to decrease or to inhibit the viral infection.

In another aspect, the present invention is directed to a method for evaluating the capacity of industrial process to eliminate or inactivate cytopathogenic agent, such as viruses or NCTA and, optionally, for the determination of their reduction factor, said method comprising the step of:

A) measuring or determining by the method according to the present invention the cytopathogenic agent titration of host cell culture infected with a sample from a liquid or fluid material, said sample being taken before and after its treatment by said industrial process;

B) Comparing the measure of the cytopathogenic agent titration of host cell culture infected for said two samples in order to determine the efficiency of said industrial process to eliminate or inactivate cytopathogenic agent susceptible to be present in said material.

In another aspect, the present invention is directed to a method for evaluating the virulence of a particular virus strain compared with a standard strain of said virus, said method comprising the step of:

A) measuring or determining by the method according to the present invention the virus titration of host cell culture infected with a sample of said particular virus strain and the virus titration of host cell culture infected with a sample of said standard virus strain, in the same conditions of culture;

B) comparing the measure obtained for these two strains of virus and determining whether said particular virus strain is more virulent than the standard strain, an increase of the titre observed for the particular viral strain tested being significant of a greater virulence of that strain compared with the standard strain.

In another aspect, the present invention encompasses a kit comprising reagents luciferin and luciferase for an ATP assay, or suitable reagents for a LDH assay, which further includes an instruction set (the instructions can be in anther embodiment provided independently of the kit). Such instructions are characterized, in part, in that they provide a user with information related to determining the viral titre of an infected host cell culture sample. Instructions can be provided in a kit, for example, written on paper or in a computer readable form provided with the kit, or can be made accessible to a user via the internet. Such instructions can, for example, instruct a user of the kit as how to obtain the standard curve in a determined condition for a control culture of virus infected host cell and thus for practicing the cell viability-based method for in-process monitoring the viral titration of cultured infected virus host cells of the present invention.

Kits of the invention may also provide instructions for practicing a method of monitoring (progression of) a cytopathogenic agent infection in a cell or in a cell culture, for screening or identifying anti cytopathogenic agent test compounds or determining the anti cytopathogenic agent efficiency of an industrial process

The following examples and also the figures and the legends hereinafter have been chosen to provide those skilled in the art with a complete description in order to be able to implement and use the present invention. These examples are not intended to limit the scope of what the inventor considers to be its invention, nor are they intended to show that only the experiments hereinafter were carried out.

## Legend of the figures

**Figures 1A and 1B:** The following graph presents the standard curve obtained with the virus MVM on A9 cell line. Tests were performed with reagents WST-I (Roche) to measure the LDH activity (Figure 1A) and celltiter glow (Promega) to measure the ATP activity (Figure 1B).

**Figure 2:** Determination of the accuracy of the new method. Titres of different stocks were compared between end point dilution assay and the new method. The following graph presents the results obtained by the two methods.

**Figure 3:** Determination of the universality of the method, dilutions of a PRV stock were performed to prove the correlation between another virus load and cell viability.

**Figures 4A and 4B:** Comparison of two titration methods for the acidic treatment evaluation on poliovirus (Figure 4A) and BVDV (Figure 4B).

## EXAMPLES

**Example 1:** Method for the determination of the viral titre

Experiments were performed in 96 wells plate. Standards from approximately $10^7$ to $10^1$ $TCID_{50}$/ml were made with 3-fold dilutions of a known virus stocks. 50 $\mu$l of the standards were added in designed wells. For unknown virus stocks, 50 $\mu$l were added in duplicate wells. Then 50 $\mu$l of a cell suspension containing $10^4$ cells were added to each wells to be tested. 96 wells plate were incubated at 37 °C with 5 % CO2 in a humidified environment. At a precise day depending on the virus tested, a cell viability test is performed to quantify the amount of a biomarker (ATP or LDH) with a plate reader (Absorbance reader, fluorimeter and luminometer).

ATP is a marker for cell viability because it's present in all metabolically active cells and the concentration declines very rapidly when the cells undergo necrosis or apoptosis. The presence of ATP in cells is determined by the production of light caused by the reaction of ATP with added luciferase and D-luciferin in the presence of $Mg^{2+}$ and molecular oxygen. Cells are currently lysed with detergents but can be lysed with other methods (heat or sonication). The emitted light is proportional to the ATP concentration and is measured using a luminometer.

The LDH biomarker reaction (Lactate dehydrogenase) is based on the cleavage of a tetrazolium salt to a formazan-class dye by mitochondrial succinate-tetrazolium reductase in viable cells. As the cells proliferate, more WST-1 is converted to the formazan product. The quantity of formazan dye is directly related to the number of metabolically active cells, and can be quantified by measuring the absorbance at 420-480 nm ($A_{max}$ 450 nm) in a multiwell plate reader. Most of the tetrazolium salt reagents are cell-permeate and don't required lysis of the cells.

A typical result shows a sigmoid curve or a polynomial curve that allow the determination of the titre between 1E+02 to 1E+06 $TCID_{50}$/ml (Figures 1A, 1B. and 3). The sigmoid function is defined as follows:

$$MES(Co)= D + (A-D) / (1 + (C0/C)B).$$

The parameter A and D describes the upper and lower limit of the sigmoid shape.

C is equal to the concentration at the turning point of the curve.

The parameter B is equivalent to the slope of the curve at the turning point of the shape.

The limit of detection is volume dependant and in this case it corresponds to 100 $TCID_{50}$/ml, or 5 $TCID_{50}$/ 50µl, or 3.5 infectious unit / tested volume that is very close to the maximal limit of detection defined as 1 infectious unit/ tested volume).

For higher titre, dilution of the stock should be done.

**Example 2:** Accuracy of the method

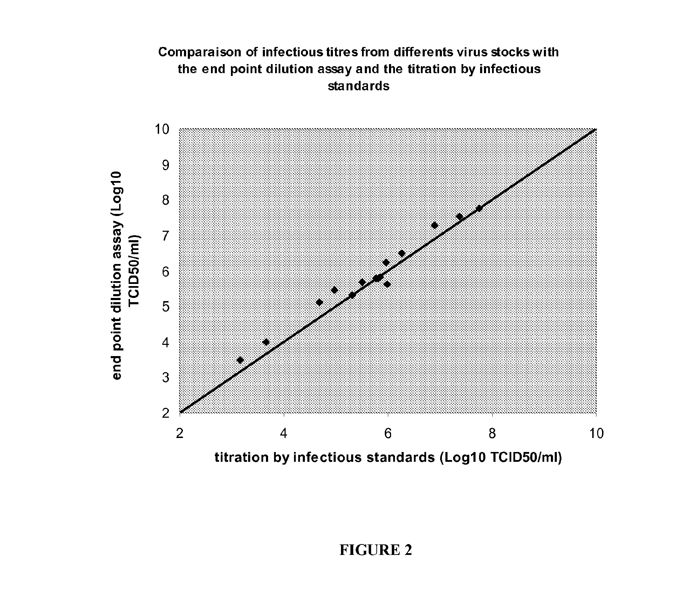

In order to demonstrate the accuracy of the new method, different MVM (murine minute virus) virus stocks were titrated both by the new methods and the end point dilution assay (Figure 2). The comparison of the titre obtained by the 2 methods show a linear relationship with titres concentrating around the straight line y=x with y defined as the end point dilution assay and x defined as the new method.

**Exemple 3:** Use of the method for viral clearance studies.

In order to demonstrate the utility of the method in viral clearance studies, we evaluate the effect of an acidic treatment on two distinct viruses (a highly resistant virus poliovirus and a virus with moderate resistance BVDV (Bovine Viral Diarrhoea Virus)).

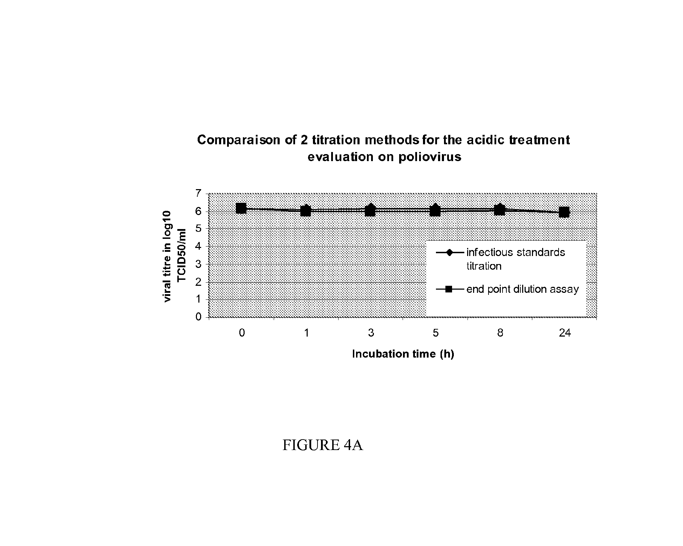

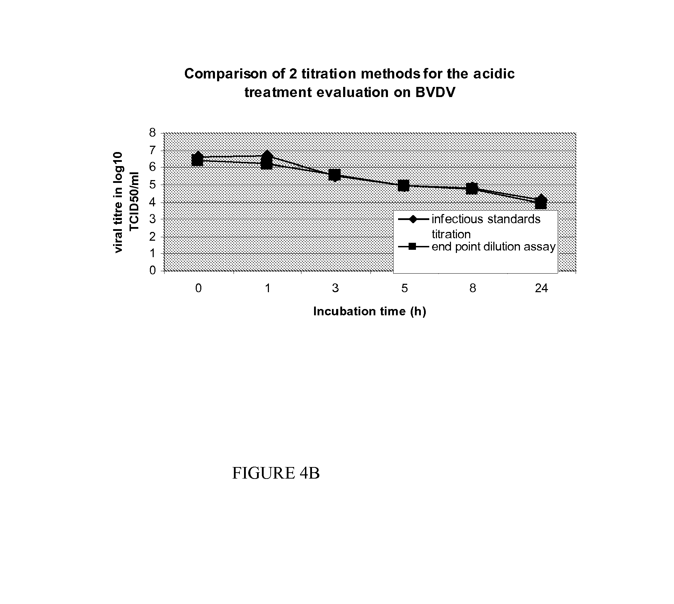

24 h time-course experiments were performed on poliovirus (Figure 4A) and BVDV (Figue 4B) in an acidic solution (pH 3). Titration were performed with the new method "titration by comparison with infectious standard" and compared with titres obtained with the dilution limit method.

Similar titres were obtained with both methods, suggesting the validity of using this new method in clearance studies.

**References:**

Cha HJ, Pham MQ, Rao G, Bentley WE.Expression of green fluorescent protein in insect larvae and its application for heterologous protein production. Biotechnol Bioeng. 1997 Nov 5;56(3):239-47.

Janakiraman V, Forrest WF, Seshagiri S.Estimation of baculovirus titer based on viable cell size.Nat Protoc. 2006; 1(5):2271-6.

Kaplan M. and Koprowski H. (Ed) in: Laboratory Techniques in Rabies. Third edition. Edited by World Health Organization, Geneva, 1973.

Kitts PA, Green G. An immunological assay for determination of baculovirus titers in 48 hours. Anal Biochem. 1999 Mar 15; 268(2):173-8. C. R. Lambeth, L. J. White, R. E. Johnston, and A. M. de Silva. Flow Cytometry-Based Assay for Titrating Dengue Virus. Journal of Clinical Microbiology, July 2005, p. 3267-3272, Vol. 43, No.
Lo HR and Chao YC. Rapid titer determination of baculovirus by quantitative real-time polymerase chain reaction. Biotechnol Prog. 2004 Jan-Feb;20(1):354-60.
Mena JA, Ramirez OT, Palomares LA. Titration of non-occluded baculovirus using a cell viability assay. Biotechniques. 2003 Feb;34(2):260-2, 264.
Pouliquen Y, Kolbinger F, Geisse S, Mahnke M. Automated baculovirus titration assay based on viable cell growth monitoring using a colorimetric indicator.Biotechniques. 2006 Mar;40(3):282, 284, 286 passim.
Schwartz D. Méthodes statistiques à l'usage des médecins et des biologistes. Flammarion Médecine-Sciences (4ème édition), 1993.
Shen CF, Meghrous J, Kamen A.Quantitation of baculovirus particles by flow cytometry.J Virol Methods. 2002 Sep; 105(2):321-30.
Thera Mulvania, Brooks Hayes and David Hedin. A Flow Cytometric Assay for Rapid, Accurate Determination of Baculovirus Titers. Bioprocess. Journal, may/june 2004. Yahata T, Andriole S, Isselbacher KJ, Shioda T.Estimation of baculovirus titer by beta-galactosidase activity assay of virus preparations.Biotechniques. 2000 Aug;29(2):214-5.

## Claims

1. A cell viability-based method for in-process monitoring the viral titration of cultured infected virus host cells, encompassing the steps of:

   a) obtaining from a host cell culture infected with a virus a concentration-dependent plurality of samples, preferably at a selected time of the cell culture;
   b) determining the level of the viability of the host cells in the samples by measuring a significant cell viability parameter; and
   c) comparing the measure of said parameter with a standard curve obtained in a same condition for a control culture of virus infected host cell.

2. A method for monitoring a viral infection or for monitoring the progression of a viral infection in a cell culture, said method comprising the step of:

   a) infecting a host cell population with virus;
   b) obtaining from said host cell culture infected with a virus a concentration-dependent plurality of samples, preferably at a selected time of the cell culture;
   c) determining the level of the viability of the host cells in the samples by measuring a significant cell viability parameter; and
   d) comparing the results or the curve obtained from said concentration-dependent plurality of samples with a standard curve obtained in a same condition for a control culture of virus infected host cell, said comparison being significant of the viral infection or its progression.

3. The method according to claim 1 or 2 wherein the standard curve is obtained in a same condition for a control culture of virus infected host cell in which the host cells are infected with virus or virus particles by contacting the host cells with a portion of a viral preparation having a known titre.

4. The method according to one of claims 1 to 3, wherein the step of determining the level of the viability of the host cells in the samples by measuring a significant cell viability parameter is determined by the measure of the intracellular ATP or the LDH activity of the host cells.

5. The method according to claim 4, wherein the step of determining the level of the viability of the host cells in the samples by measuring a significant cell viability parameter is determined by the measure of the intracellular ATP after the lysis of the virus infected host cells of the sample.

6. The method according to claim 5, wherein the intracellular ATP is determined by bio luminescence.

7. The method according to one of claims 1 to 6, wherein said host cells are cells selected from the group consisting of cell lines or primary cells that are permissive to virus infection.

8. The method according to one of claims 1 to 7, wherein said host cells are cells selected from the group consisting of animal cells, preferably mammalian cells

9. The method according to one of claims 1 to 8, wherein said host cells are cells selected from the group consisting of RK-13 and A9 cell lines.

10. The method according to one of claims 1 to 9, wherein said virus or virus particles are selected from the group consisting of any viruses that lead to a cytopathogenic effect in a cell monolayer.

11. The method according to one of claims 1 to 10, wherein said virus or virus particles are selected from the group consisting of adenovirus, herpes simplex virus, parvovirus, Epstein Barr virus, parainfluenza virus, parvovirus, bovine viral diarrhea virus, sindbis virus, baculovirus, pseudorabies virus, hepatitis A virus, West nile virus and vaccinia virus.

12. A method for the screening, the identification or the optimization of antiviral test compounds involved in drug development and/or in pharmaceutical compositions, said method comprising the step of:

A) Monitoring the viral titration of cultured infected virus host cells, by the method according to one of claims 1 to 11, wherein the viral titration is carried out in presence and in absence of the antiviral test compound;
B) Comparing the measure of the viral titration obtained and determining whether this antiviral test compound has the capacity to decrease or to inhibit the viral infection.

13. A method for studies for evaluating the capacity of industrial process to eliminate or inactivate viruses and for the determination of their reduction factor,
said method comprising the step of:

A) measuring or determining by the method according to one of claims 1 to 11, the viral titration of host cell culture infected with a sample from a liquid, vapour or solid material, said sample being taken before and after its treatment by said industrial process;
B) Comparing the measure of the viral titration of host cell culture infected for said two samples in order to determine the efficiency of said industrial process to eliminate or inactivate viruses susceptible to be present in said material.

14. A method for evaluating the virulence or a particular virus strain compared with a standard strain of said virus, said method comprising the step of:

A) measuring or determining by the method according to one of claims 1 to 11, the virus titration of host cell culture infected with a sample of said particular virus strain and the virus titration of host cell culture infected with a sample of said standard virus strain, in the same conditions of culture;
B) comparing the measure of the titration obtained for these two strains of virus and determining whether said particular virus strain is more virulent than the standard strain, an increase of the titre observed for the particular viral strain tested being significant of a greater virulence of that strain compared with the standard strain.

**FIGURE 1A**

**FIGURE 1B**

Comparaison of infectious titres from differents virus stocks with the end point dilution assay and the titration by infectious standards

FIGURE 2

**FIGURE 3**

Comparaison of 2 titration methods for the acidic treatment evaluation on poliovirus

FIGURE 4A

**Comparison of 2 titration methods for the acidic treatment evaluation on BVDV**

FIGURE 4B

Europäisches
Patentamt

European
Patent Office

Office européen
des brevets

# EUROPEAN SEARCH REPORT

Application Number

EP 10 30 6347

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PETER T. WITKOWSKI ET AL: "Cellular impedance measurement as a new tool for poxvirus titration, antibody neutralization testing and evaluation of antiviral substances", BIOCHEMICAL AND BIOPHYSICAL RESEARCH COMMUNICATIONS, vol. 401, no. 1, 8 October 2010 (2010-10-08), pages 37-41, XP55000087, ISSN: 0006-291X, DOI: 10.1016/j.bbrc.2010.09.003 * abstract * * paragraphs [02.1], [02.3], [02.4], [03.1], [03.2], [0004] * * figure 1 * | 1-14 | INV. G01N33/569 |
| X | US 2008/233561 A1 (WANG EUGENIA [US]) 25 September 2008 (2008-09-25) * abstract * * paragraphs [0003], [0012], [0035] - [0039], [0062], [0063] * * figure 2 * * claims 1,7 * | 1,2,7,8, 10,11 | |
| X | National RNAi Core Facility: "Relative Viral Titering Using Cell Viability Assay (RIU Method)", , 22 January 2008 (2008-01-22), XP002638320, Retrieved from the Internet: URL:http://dfpcorec-p/wf/web/citenpl/citen pl.html?_url=http%3A%2F%2Frnai.genmed.sini ca.edu.tw%2Ffile%2Fprotocol%2F4_1_Estimati onLentivirusTiterRIUV1.pdf [retrieved on 2011-05-23] * the whole document * | 1-3,7,8, 10 | |

TECHNICAL FIELDS SEARCHED (IPC)

G01N

-/--

The present search report has been drawn up for all claims

| Place of search | Date of completion of the search | Examiner |
|---|---|---|
| The Hague | 25 May 2011 | Adida, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**Application Number**

EP 10 30 6347

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2010/078229 A1 (SOUTHERN RES INST [US]; MADDRY JOSEPH A [US]; SEVERSON WILLIAM [US]; J) 8 July 2010 (2010-07-08) * paragraph [0093] * ----- | 1-14 | |
| A | MORI S ET AL: "A colorimetric LDH assay for the titration of infectivity and the evaluation of anti-viral activity against ortho- and paramyxoviruses.", THE TOHOKU JOURNAL OF EXPERIMENTAL MEDICINE DEC 1995 LNKD- PUBMED:8928191, vol. 177, no. 4, December 1995 (1995-12), pages 315-325, XP002638266, ISSN: 0040-8727 * the whole document * ----- | 1-14 | |
| A | J O GOLUB: "A single-dilution method for the estimation of LD50 titers of the psittacosis-LGV group of viruses in chick embryos.", J Immunol., 1948, pages 71-82, XP55000091, Retrieved from the Internet: URL:http://www.jimmunol.org/content/59/1/71.full.pdf#page=1&view=FitH [retrieved on 2011-05-23] * the whole document * ----- | 1-14 | **TECHNICAL FIELDS SEARCHED** (IPC) |
| A | Jean M Riley ET AL: "Single-Dose Assay Technique for Variola Virus", Appl. Envir. Microbiol., 1964, pages 7-9, XP55000093, Retrieved from the Internet: URL:http://aem.asm.org/cgi/reprint/12/1/7.pdf [retrieved on 2011-05-23] * the whole document * ----- | 1-14 | |

-/--

The present search report has been drawn up for all claims

| Place of search | Date of completion of the search | Examiner |
|---|---|---|
| The Hague | 25 May 2011 | Adida, Anne |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 10 30 6347

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2006/195268 A1 (VEGA MANUEL [FR]) 31 August 2006 (2006-08-31) * the whole document * ----- | 1-14 | |

TECHNICAL FIELDS
SEARCHED      (IPC)

The present search report has been drawn up for all claims

| Place of search | Date of completion of the search | Examiner |
|---|---|---|
| The Hague | 25 May 2011 | Adida, Anne |

EPO FORM 1503 03.82 (P04C01)

**ANNEX TO THE EUROPEAN SEARCH REPORT
ON EUROPEAN PATENT APPLICATION NO.**

EP 10 30 6347

This annex lists the patent family members relating to the patent documents cited in the above-mentioned European search report.
The members are as contained in the European Patent Office EDP file on
The European Patent Office is in no way liable for these particulars which are merely given for the purpose of information.

25-05-2011

| Patent document cited in search report | | Publication date | Patent family member(s) | | Publication date |
|---|---|---|---|---|---|
| US 2008233561 | A1 | 25-09-2008 | WO | 2007015878 A2 | 08-02-2007 |
| WO 2010078229 | A1 | 08-07-2010 | NONE | | |
| US 2006195268 | A1 | 31-08-2006 | AT | 384263 T | 15-02-2008 |
| | | | EP | 1281081 A1 | 05-02-2003 |
| | | | FR | 2808804 A1 | 16-11-2001 |
| | | | WO | 0186291 A1 | 15-11-2001 |
| | | | US | 2003175694 A1 | 18-09-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHA HJ ; PHAM MQ ; RAO G.** Bentley WE.Expression of green fluorescent protein in insect larvae and its application for heterologous protein production. *Biotechnol Bioeng.,* 05 November 1997, vol. 56 (3), 239-47
- **JANAKIRAMAN V ; FORREST WF ; SESHAGIRI S.** Estimation of baculovirus titer based on viable cell size. *Nat Protoc.,* 2006, vol. 1 (5), 2271-6
- Laboratory Techniques in Rabies. 1973
- **KITTS PA ; GREEN G.** An immunological assay for determination of baculovirus titers in 48 hours. *Anal Biochem.,* 15 March 1999, vol. 268 (2), 173-8
- **C. R. LAMBETH ; L. J. WHITE ; R. E. JOHNSTON ; A. M. DE SILVA.** Flow Cytometry-Based Assay for Titrating Dengue Virus. *Journal of Clinical Microbiology,* July 2005, vol. 43, 3267-3272
- **LO HR ; CHAO YC.** Rapid titer determination of baculovirus by quantitative real-time polymerase chain reaction. *Biotechnol Prog.,* January 2004, vol. 20 (1), 354-60
- **MENA JA ; RAMIREZ OT ; PALOMARES LA.** Titration of non-occluded baculovirus using a cell viability assay. *Biotechniques,* February 2003, vol. 34 (2), 260-2264
- **POULIQUEN Y ; KOLBINGER F ; GEISSE S ; MAHNKE M.** Automated baculovirus titration assay based on viable cell growth monitoring using a colorimetric indicator. *Biotechniques,* March 2006, vol. 40 (3), 282, 284, 286
- **SCHWARTZ D.** Méthodes statistiques à l'usage des médecins et des biologistes. Flammarion Médecine-Sciences, 1993
- **SHEN CF ; MEGHROUS J ; KAMEN A.** Quantitation of baculovirus particles by flow cytometry. *J Virol Methods,* September 2002, vol. 105 (2), 321-30
- **THERA MULVANIA ; BROOKS HAYES ; DAVID HEDIN.** A Flow Cytometric Assay for Rapid, Accurate Determination of Baculovirus Titers. Bioprocess. *Journal,* May 2004
- **YAHATA T ; ANDRIOLE S ; ISSELBACHER KJ ; SHIODA T.** Estimation of baculovirus titer by beta-galactosidase activity assay of virus preparations. *Biotechniques,* August 2000, vol. 29 (2), 214-5